# FASCICULE DE BREVET EUROPEEN

(11) **EP 3 323 386 B1**
(45) Date de publication et mention de la délivrance du brevet: **15.05.2019**
(21) Numéro de dépôt: 17201759.2
(22) Date de dépôt: 15.11.2017
(51) Int. Cl.: A61F 2/08

(54) **DISPOSITIF DE FIXATION IMPLANTABLE POUR LA SOLIDARISATION D'UN GREFFON A AU MOINS UN OS D'UNE ARTICULATION**
IMPLANTIERBARE BEFESTIGUNGSVORRICHTUNG FÜR DIE VERBINDUNG EINES TRANSPLANTATS MIT MINDESTENS EINEM KNOCHEN EINES GELENKS
IMPLANTABLE ATTACHMENT DEVICE FOR SECURING A GRAFT TO AT LEAST ONE BONE OF A JOINT

(30) Priorité: 21.11.2016 FR 1661308
(43) Date de publication de la demande: 23.05.2018
(73) Titulaire: Cousin Biotech, 59117 Wervicq Sud (FR)
(72) Inventeur: PRANDI, Jules, 69100 Villeurbanne (FR); KAMCHE, Farid, 59200 Tourcoing (FR)
(74) Mandataire: Cabinet Beau de Loménie

(56) Documents cités:
- EP-A1- 2 666 441
- FR-A1- 2 980 356

## Description

### Arrière-plan de l'invention

La présente invention concerne le domaine technique des dispositifs de fixation implantable pour la solidarisation d'un greffon à au moins un os d'une articulation, en particulier à au moins un os de l'articulation du genou pour la reconstruction du ligament croisé antérieur ou postérieur.

Les dispositifs de fixation implantables pour la reconstruction du Ligament Croisé Antérieur (LCA) ou du Ligament Croisé Postérieur (LCP) mettent en oeuvre un greffon confectionné sur mesure. Ce greffon est généralement confectionné avec le tendon « droit interne » (DI) ou « gracile », et le tendon « demi-tendineux » (DT). La technique opératoire associée à cette préparation du greffon porte le nom de technique « DIDT ».

La technique opératoire ne consistant à utiliser que le tendon « demi-tendineux » plié en quatre porte le nom de « DT4 ».

La technique opératoire utilisant la partie centrale du tendon rotulien porte le nom de « Kenneth Jones » ou « KJ » selon le nom du chirurgien qui l'a mise au point.

Toutes ces méthodes de traitement chirurgicales comprennent le placement d'un greffon constitué d'un ou plusieurs tendons, sous contrôle arthroscopique, dans un tunnel osseux réalisé par forage dans la tête du tibia et la tête du fémur.

Le « droit interne » et le « demi-tendineux » sont deux muscles semblables, qui avec un troisième muscle, le « couturier », forment « la patte d'oie ». Ils sont également appelés muscles « ischiojambiers » et sont disposés sur la partie interne de la cuisse. Les tendons à ce niveau de la cuisse sont très longs et sont attachés à la face interne du tibia après avoir croisé le genou. Il est possible de prélever ces tendons par une petite incision de quelques centimètres à l'aide d'un instrument du type « stripper » qui les détache sur toute leur longueur.

Une fois les tendons prélevés, ils sont joints et pliés en deux ce qui permet de former un nouveau ligament appelé greffon ou autogreffe, constitué de quatre brins dont la résistance à la traction est ainsi supérieur à celle d'un Ligament Croisé Antérieur (LCA) normal.

Les tunnels fémoral et tibial réalisés par forage permettent de faire passer le greffon à l'intérieur de l'articulation afin de le disposer à l'endroit où était situé l'ancien ligament croisé antérieur. Les tunnels fémoral et tibial débouchent à l'endroit des attaches naturelles du ligament. Le positionnement de ces tunnels tibial et fémoral est important car il influe sur la stabilité post-opératoire ultérieure du genou. Le tunnel fémoral est effectué directement à l'intérieur de l'articulation par l'un des deux orifices de l'arthroscopie ou par le tunnel tibial préalablement foré. Le tunnel fémoral peut également être foré par l'intermédiaire d'une petite incision effectuée selon la face externe de la cuisse.

La fixation du greffon se fait à l'aide de dispositifs médicaux aux extrémités des tunnels fémoral et tibial perforés.

On connaît des dispositifs de fixation implantables, utilisés en particulier dans les techniques opératoires DIDT et DT4, comprenant des moyens de fixation des extrémités du greffon à l'articulation, lesdits moyens de fixation sont par exemple une vis d'interférence introduite dans la paroi du tunnel comprenant le greffon et venant ainsi bloquer le greffon contre la paroi du tunnel.

La fixation du greffon peut être effectuée également en fixant à l'une extrémité ou aux deux extrémités du greffon, une bande textile, les moyens de fixation étant alors appliqués directement aux bandes textiles et non au greffon. Un autre moyen de fixation comprend une cage disposée dans un tunnel et ayant un alésage intérieur apte à coopérer avec le pas de vis d'une vis de blocage, la bande textile étant bloquée dans la cage par la vis de blocage vissée.

Ce type de dispositifs ne permet pas l'ajustement de la position du greffon dans les tunnels fémoral et tibial après sa mise en place sans une reprise chirurgicale.

WO 2012/154922 a pour objet un dispositif implantable comprenant un élément longiligne creux dont au moins une portion est passée est à travers deux ouvertures traversantes d'un bouton d'appui, une première extrémité de l'élément longiligne est passée à l'intérieur de l'élément longiligne dans sa seconde extrémité puis ressort sur la longueur de celui-ci en sorte de forme une boucle de serrage. En fonctionnement, l'ajustement de la taille de la boucle de serrage est obtenu en exerçant une traction sur la première extrémité libre de l'élément longiligne, laquelle première extrémité s'étend au moins dans le canal fémoral et vient donc en contact avec le greffon. Il y a ainsi un risque d'abraser et donc d'endommager le greffon et modifier sa position dans le canal fémoral, et éventuellement dans le canal tibial si la première extrémité s'étend également dans ce canal.

FR 2 980 356 a pour objet un dispositif comprenant un élément longiligne pourvu, selon deux zones de sa longueur, d'au moins deux passants recevant chacun une extrémité de l'élément longiligne en sorte de former deux boucles de serrage. Une portion de l'élément longiligne est passée à travers deux ouvertures traversantes d'un bouton d'appui.

Comparativement à WO 2012/154922, les deux extrémités libres de FR 2 980 356 s'étendent de façon opposée aux deux boucles de serrage, ce qui évite que les deux extrémités libres s'étendent à travers le canal fémoral, et éventuellement le canal tibial. Cependant, une traction exercée sur les extrémités libres de l'élément longiligne ne permet que de régler la taille de boucles en liaison avec le greffon. Le dispositif n'est pas pourvu de moyens de verrouillage du greffon dans sa position d'implantation puisque, si une tension est exercée sur le greffon à travers le canal fémoral, et éventuellement le canal tibial, les brins de l'élément longiligne disposés dans les passants coulissent, les boucles s'agrandissent et le greffon n'est plus correctement mis en tension, et est bougé de sa position d'implantation.

Il existe donc un besoin pour un dispositif de fixation implantable d'un greffon à au moins un os d'une articulation permettant de régler la position du greffon dans au moins un canal osseux, en particulier le canal fémoral et le canal tibial, de verrouiller, et ce de façon amovible sans reprise chirurgicale, le greffon dans ledit canal osseux, en particulier le canal fémoral et le canal tibial ; et de moyens pour régler la position et la mise en tension du greffon, et ce de façon amovible.

Il existe également un besoin pour un dispositif de fixation implantable simple à mettre en oeuvre et limitant les frottements exercés sur le greffon afin de préserver les propriétés mécaniques de ce dernier.

### Objet et résumé de l'invention

La présente invention pallie les problèmes précités en ce qu'elle a pour objet un dispositif de fixation implantable pour la solidarisation d'un greffon à au moins un os d'une articulation, par exemple à l'os tibial et/ou à l'os fémoral, notamment pour la reconstruction du ligament croisé antérieur ou postérieur du genou, ledit dispositif de fixation comprenant :
- un élément textile longiligne tubulaire creux délimitant un volume intérieur et comprenant une première extrémité libre et une seconde extrémité libre, et
- un élément d'appui comprenant une face d'appui configurée pour venir en appui contre au moins ledit os d'une articulation, ledit élément d'appui comprenant au moins deux ouvertures traversantes.

Avantageusement, ledit élément longiligne a un diamètre externe initial d0 (mm) et comprend un orifice d'admission dans son volume intérieur et un orifice de sortie de son volume intérieur distants d'une distance initial m0 (mm) délimitant un manchon, la première extrémité dudit élément longiligne étant passée à travers lesdits orifices d'admission et de sortie, dans le manchon, en sorte de former une première boucle, de périmètre initial P1 ajustable, dont au moins une portion est passée à travers au moins deux ouvertures traversantes dudit élément d'appui. La première extrémité libre dudit élément longiligne passe à travers une ouverture traversante dudit élément d'appui, et ladite seconde extrémité libre passe également à travers une ouverture traversante dudit élément d'appui et forme une deuxième boucle de périmètre initial P2 ajustable. Le manchon, recevant une portion d'élément longiligne, a un diamètre externe initial n0, et la distance m0 et le diamètre externe d0 dudit élément longiligne sont déterminés en sorte qu'en fonctionnement, une traction exercée sur la première extrémité libre provoque le rapprochement des orifices d'admission et de sortie et corrélativement l'expansion radiale du manchon.

Le dispositif implantable selon l'invention forme ainsi deux boucles de périmètre ajustable, aptes chacune à recevoir une boucle d'un ligament et/ou d'un tendon.

Avantageusement, l'expansion radiale du manchon engendre un diamètre n1 (à l'état comprimé) supérieur au diamètre n0 initiale du manchon ce qui permet l'immobilisation du dispositif implantable dans ledit au moins un canal osseux dans lequel le dispositif est disposé. La distance m0, et le diamètre externe d0 de l'élément longiligne sont déterminées l'un par rapport à l'autre en sorte que la traction exercée sur la première extrémité libre de l'élément longiligne engendre du frottement entre la surface externe de l'élément longiligne et la surface interne du manchon provoquant ainsi sa compression radiale et donc le rapprochement des orifices d'admission et de sortie. La distance m1 séparant les orifices d'admission et de sortie du manchon dans son état comprimé est inférieure à la distance initiale m0 séparant lesdits orifices d'admission et de sortie du manchon.

Encore avantageusement, la traction sur la première extrémité libre dudit élément longiligne permet également le réglage du périmètre de la première boucle par coulissement de l'élément longiligne dans le manchon à l'état comprimé et à travers les ouvertures traversantes dudit élément d'appui. Le périmètre initiale P1 de la première boucle est ainsi réduit lorsqu'une traction est exercée sur la première extrémité libre de l'élément longiligne permettant ainsi le réglage de la position du tendon ou du ligament dans ledit au moins un canal osseux.

Il est possible de replacer le manchon dans son état initial, c'est-à-dire non comprimé dans lequel la distance séparant les orifices d'admission et de sortie est m0, en exerçant une traction sur l'élément longiligne dans une direction opposée à la traction appliquée pour obtenir la compression du manchon et en maintenant le manchon afin qu'il ne s'allonge pas. Le dispositif implantable comprend ainsi avantageusement des moyens d'immobilisation et de réglage de la position du greffon passé dans les première et seconde boucles qui sont amovibles.

Dans un mode de réalisation, la distance m0 du manchon est supérieure ou égale à cinq fois, de préférence supérieure ou égale à 10 fois, encore de préférence supérieure ou égale à douze fois, encore plus préférentiellement supérieure ou égale à quinze fois le diamètre externe d0 de l'élément longiligne.

La mesure des diamètres et des distances sur le dispositif selon l'invention, telles que les distances m0, m1 ou m2, peuvent être mesurées à l'aide d'un pied-à-coulisse ou encore par exemple à l'aide d'un support graduée et d'une loupe. Les distances et diamètres sont indiqués en millimètre (mm) dans le cadre de la présente invention.

Dans un mode de réalisation, la distance m0 est supérieure ou égale à 25 mm et inférieure ou égale à 50 mm, de préférence supérieure ou égale à 30 mm et inférieure ou égale à 45 mm.

De préférence, le diamètre externe initial n0 du manchon (état initial) est supérieur ou égal à 1,50 mm et inférieur ou égal à 3 mm, encore de préférence supérieur ou égal à 2,00 mm et inférieur ou égal à 2,80 mm, notamment de l'ordre de 2,50 mm.

Dans un mode de réalisation, la distance m1 est supérieure ou égale à 15 mm et inférieure ou égale à 35 mm, de préférence supérieure ou égale à 20 mm et inférieure ou égale à 35 mm.

De préférence, le diamètre externe n1 du manchon (état comprimé) est supérieur ou égal à 1,5 mm et inférieur ou égal à 4 mm, encore de préférence supérieur ou égal à 2 et inférieur ou égal à 3,5 mm.

De préférence, le diamètre externe d0 de l'élément longiligne tubulaire est supérieur ou égal à 1,30 mm et inférieur ou égal à 3 mm, encore de préférence supérieur ou égal à 1,50 mm et inférieur ou égal à 2,50 mm.

Dans un mode de réalisation, ladite au moins une portion de l'élément longiligne passe à travers une première ouverture traversante et une seconde ouverture traversante de l'élément d'appui, et la première extrémité libre de l'élément longiligne passe à travers ladite première ouverture traversante, tandis que la seconde extrémité libre passe à travers ladite seconde ouverture traversante.

De préférence, les première et seconde extrémités libres de l'élément longiligne débouchent de l'élément d'appui selon sa face externe opposée à la face d'appui, tandis que les première et seconde boucles se projettent de la face d'appui. Cette disposition permet d'éviter les frottements des première et seconde extrémités libres de l'élément longiligne sur le greffon, puisque lesdites extrémités libres ne s'étendent pas le long dudit au moins un canal osseux, et ne viennent pas ainsi coulisser en abrasant le greffon.

Dans un mode de réalisation, l'élément d'appui se présente sous la forme d'un bouton comprenant au moins deux ouvertures traversantes ayant une face d'appui apte à venir en appui sur un élément osseux, et une face externe, opposée à la face d'appui.

L'élément d'appui peut être dans n'importe quel matériau implantable, et notamment dans un alliage de titane et de nickel, par exemple le nitinol ; ou encore dans un alliage de titane et d'aluminium, tel que celui connu sous la référence Ti 6AI-4V qui est un alliage de titane comportant 6 % en masse d'aluminium et 4 % en masse de vanadium par rapport à la masse totale dudit alliage, le restant étant de l'aluminium ; ou encore dans un matériau synthétique, de préférence choisi parmi les matériaux synthétiques suivants : le polyéthylènetéréphtalate ; le polyamide 6 ; le polyétherétherkétone (PEEK), notamment de marque PEEK-OPTIMA®, ou encore de marque OXPEKK® ; le polyéthylène de haut poids moléculaire portant la désignation UHMWPE pour « Ultra High Molecular Weight Polyethylene », par exemple de marque GUR®.

De préférence, la première extrémité libre et la seconde extrémité libre dudit élément longiligne se projettent selon la face externe dudit élément d'appui, qui est disposée de manière opposée à la face d'appui dudit élément d'appui.

L'élément longiligne textile est souple du fait de structure textile. L'élément longiligne textile pourrait être dans n'importe quel élément textile : tissé, tricoté ou tressé. De préférence, les meilleurs résultats en termes de compression et d'allongement du manchon, ont été obtenus lorsque l'élément longiligne est une tresse.

L'élément longiligne textile comprend des fils filés de fibres et/ou des fils multifilamentaires, de préférence des fils multifilamentaires.

Lesdits fils filés de fibres et/ou fils multifilamentaires sont de préférence choisis dans la liste comprenant : les polyoléfines, en particulier les polyéthylènes UHMWPE, notamment de marque DYNEEMA® ou de marque SPECTRA®, ou le polypropylène ; le polytétrafluoroéthylène (PTFE) ; les polyamides, en particulier le polyamide 6 ou le polyamide 6-6 ; les polyesters, en particulier le polyéthylènetéréphtalate.

Les fils filés de fibres et/ou fils multifilamentaires sont de préférence dans un ou plusieurs matériau(x) non résorbable(s), tels que ceux cités ci-dessus.

Le dispositif implantable selon l'invention, bien qu'étant préféré pour la reconstruction du ligament croisé antérieur ou du ligament croisé postérieur du genou, n'est pas limité à cette application.

Le dispositif implantable selon l'invention peut ainsi être utilisé de manière générale dans le domaine de la réinsertion tendineuse ou ligamentaire au niveau de l'épaule, de la hanche, du genou ou par exemple de la cheville afin de réparer des ruptures, des instabilités, des disjonctions comme la disjonction acromio-claviculaire, ou pour la réalisation de ténodèse (la fixation d'un tendon sur un autre tendon ou bien sur un os, dans le cadre de la présente invention la fixation du tendon est effectuée sur au moins un os).

On entend par greffon selon l'invention, tout ligament ou tendon ou autre matériau implantable équivalent, il peut s'agir par exemple d'une autogreffe ou d'une allogreffe ; de préférence il s'agit d'une autogreffe.

Dans une variante, le périmètre initial P2 est supérieur au périmètre initial P1, de préférence le périmètre initial P2 est supérieur ou égal à 1,5 fois le périmètre initial P1.

De préférence, le périmètre initial P1 est supérieur ou égal à 75 mm et inférieur ou égal à 300 mm, encore de préférence supérieur ou égal à 105 mm et inférieur ou égal à 150 mm.

De préférence, le périmètre initial P2 est supérieur ou égal à 0 mm et inférieur ou égal à 200 mm, encore de préférence supérieur ou égal à 70 mm et inférieur ou égal à 100 mm.

Dans une variante, l'orifice de sortie du manchon vient en butée contre une ouverture traversante dudit élément d'appui lorsqu'en fonctionnement, une traction est exercée sur ladite première extrémité libre.

Cette disposition permet avantageusement d'améliorer l'effet d'immobilisation du dispositif implantable et en particulier du manchon dans le canal osseux dans lequel il est disposé.

Dans une variante, lorsqu'une traction est exercée sur la seconde extrémité libre dudit d'élément longiligne, les orifices d'admission et de sortie s'écartent pour atteindre une distance m2 correspondant à une position de verrouillage dans laquelle la portion d'élément longiligne disposée dans ledit manchon est bloquée, en particulier la distance m2 est supérieure à la distance initiale m0 séparant lesdits orifices d'admission et de sortie.

Dans un mode de réalisation, la distance m2 du manchon (état allongé) est supérieure ou égale à 30 mm et inférieure ou égale à 50 mm, encore de préférence supérieure ou égale à 35 mm et inférieure ou égale à 45 mm.

De préférence, le diamètre externe n2 du manchon (état allongé), est de l'ordre de 2,00 mm.

Avantageusement, la traction sur la seconde extrémité libre de l'élément longiligne permet corrélativement le réglage du périmètre de la seconde boucle du dispositif et donc le réglage de la position du tendon et/ou du ligament passé dans la dite seconde boucle mais permet également de bloquer le coulissement de l'élément longiligne dans le manchon. En effet, les frottements exercées entre la surface externe de l'élément longiligne et la surface interne du manchon selon une seconde direction (qui est opposée à la première direction de traction sur la première extrémité de l'élément longiligne et permettant la compression du manchon) obtenue par traction sur la seconde extrémité libre de l'élément longiligne permet l'éloignement de l'orifice d'admission de l'orifice de sortie et donc l'allongement du manchon à une distance m2 supérieure à la distance m0 (état initial) et à la distance m1 (état comprimé).

Dans une variante, une traction sur la seconde extrémité libre entraîne la diminution du périmètre P2 de la seconde boucle.

Dans une variante, la première boucle est disposée à l'intérieur, au moins en partie, de la deuxième boucle, et en ce que les première et seconde boucles s'étendent de la face d'appui dudit élément d'appui.

Dans une variante, l'élément tubulaire longiligne est une tresse.

Dans une variante, l'élément tubulaire est une tresse de structure perlée.

Cette structure perlée est également désignée dans l'état de la technique par structure en diamant.

Cette structure permet d'obtenir les résultats optimaux en termes de frottements générés entre la surface externe de l'élément longiligne et la surface interne du manchon.

Dans une variante, l'élément tubulaire longiligne est une tresse comprenant un premier groupe de mèches tressées et un second groupe de mèches tressées, le premier groupe de mèches a une torsion Z tandis que le second groupe de mèches a une torsion S.

Cette disposition est de préférence obtenue en disposant chaque mèche du premier groupe de mèches sur un fuseau tournant dans le sens Z tandis que chaque mèche du second groupe de mèches est disposée sur un fuseau tournant dans le sens S.

De préférence, le premier groupe de mèches comprend un nombre de mèches de l'ordre de 0,8 à 1,2 fois, encore de préférence de l'ordre de 0,9 à 1,1 fois, le nombre de mèches du second groupe de mèches. Cette disposition permet d'obtenir un montage équilibré entre les premiers et second groupes de mèches et un frottement optimal entre la surface interne du manchon et la surface externe de l'élément longiligne.

Dans une variante, l'élément tubulaire longiligne est une tresse comprenant un premier groupe de mèches dont chaque mèche forme un angle alpha délimité entre l'axe longitudinal L de l'élément tubulaire longiligne tressé et l'axe transversal T, sensiblement perpendiculaire à l'axe L, supérieur ou égal à (+)10° et inférieur ou égal à (+)90°, et un second groupe de mèches dont chaque mèche forme un angle beta délimité entre l'axe longitudinal L de l'élément tubulaire longiligne tressé et l'axe transversal T, sensiblement perpendiculaire à l'axe L, supérieur ou égal à (+)100° et inférieur ou égal à (+)180°.

L'expression (+) indique que l'angle est mesuré selon le sens trigonométrique.

Le demandeur s'est aperçu que cette disposition permet d'obtenir des résultats optimaux en termes de frottements entre le manchon et l'élément longiligne et de résistance à rupture.

L'élément longiligne selon l'invention étant destiné à rester implanté et étant soumis à de nombreuses contraintes mécaniques en particulier lorsqu'il est implanté au niveau d'une articulation (traction, cisaillement, flexion,...), il doit présenter une résistance à rupture suffisante.

L'élément longiligne tubulaire selon l'invention a une résistance à la rupture supérieure ou égale à 500 Newtons, notamment de l'ordre de 800 Newtons, et un allongement à la rupture inférieur ou égal à 15%, en particulier de l'ordre de 5%.

Dans une variante, le premier groupe de mèches, et éventuellement le second groupe de mèches, comprend/comprennent, au moins 4 mèches, encore de préférence au moins 8 mèches, en particulier au moins 12 mèches, plus particulièrement au moins 16 mèches.

Dans une variante, chaque mèche du premier groupe de mèches, et éventuellement du second groupe de mèches, comprend/comprennent au moins 4 fils, notamment au moins 6 fils, retordus selon un nombre de tours/mètre compris entre 100 et 300.

De préférence, le ou lesdits fils sont des fils filés de fibres et/ou des fils multifilamentaires, encore de préférence des fils multifilamentaires tels que définis ci-avant en référence à l'élément longiligne tubulaire.

Dans une variante, l'élément tubulaire longiligne textile comprend des fils ayant un titre supérieur ou égal à 100 dtex et inférieur ou égal à 300 dtex.

### Brève description des dessins

La présente invention sera mieux comprise à la lecture de la description qui suit d'un mode de réalisation de l'invention, donné à titre d'exemple non limitatif, et illustré par les figures suivantes dans lesquelles :
- la figure **1** est une représentation schématique d'un exemple de dispositif implantable selon l'invention dans son état initial ;
- la figure **2** est une représentation schématique de l'exemple de dispositif implantable représenté à la figure **1** dans lequel le manchon est dans son état comprimé correspondant à une position d'immobilisation dudit dispositif;
- la figure **3** est une représentation schématique de l'exemple de dispositif implantable représenté aux figures **1** et **2** dans lequel le manchon est dans son état allongé correspondant à une position de verrouillage dudit dispositif;
- la figure **4** est une représentation schématique du motif de tressage en diamant ou perlé utilisé pour l'élément longiligne du dispositif implantable représenté sur les figures **1** à **3** ;
- la figure **5** est une représentation schématique des angles formés par les premier et second groupes de mèches tressés formant l'élément longiligne du dispositif implantable représenté aux figures **1** à **3** ; et
- la figure **6** est une représentation schématique représentant le dispositif à la figure **1** dans laquelle le canal anatomique a une morphologie différente de celle représentée à la figure **1****.**

### Description détaillée de l'invention

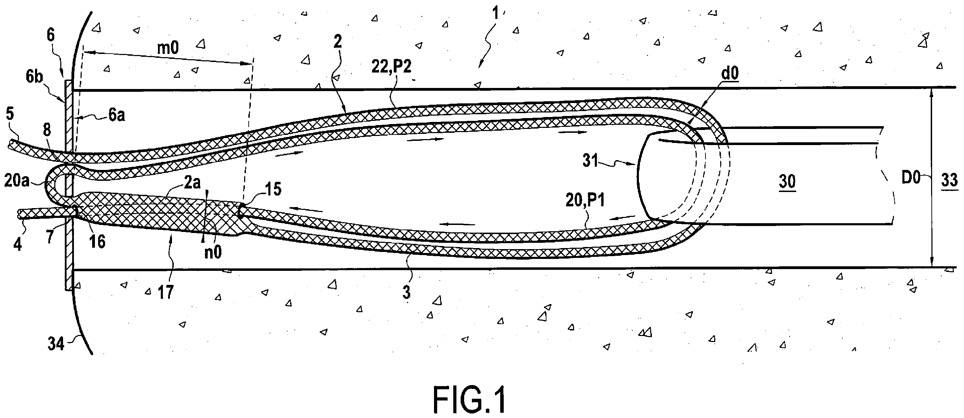

Le dispositif de fixation implantable **1** représenté à la figure **1** est de préférence destiné à la solidarisation d'un greffon à au moins un os d'une articulation, notamment à l'os tibial et/ou à l'os fémoral pour la reconstruction du ligament croisé antérieur ou postérieur du genou. Cette application bien que préférée n'est cependant pas limitative du dispositif implantable **1** décrit ci-après.

Le dispositif implantable **1** comprend un élément textile longiligne tubulaire **2** creux délimitant un volume intérieur **3** et comprenant une première extrémité libre **4** et une seconde extrémité libre **5,** ainsi qu'un élément d'appui **6** comprenant une face d'appui **6a** configurée pour venir en appui contre au moins un os d'une articulation, ledit élément d'appui **6** comprenant au moins deux ouvertures traversantes, en particulier une première **7** et une seconde **8** ouvertures traversantes. L'élément d'appui **6** comprend une face externe **6b,** opposée à la face d'appui **6a.** De préférence, l'élément d'appui **6** est un bouton.

La figure **1** représente l'élément longiligne **2** dans son état initial. L'élément longiligne **2** présente dans cet état initial un diamètre externe initial **d0** (mm), par exemple de l'ordre de 2,30 mm, et comprend un orifice d'admission **15** dans son volume intérieur et un orifice de sortie **16** de son volume intérieur distants d'une distance initial **m0** (mm) délimitant un manchon **17.** La première extrémité **4** dudit élément longiligne **2** est passée à travers lesdits orifices d'admission **15** et de sortie **16,** dans le manchon **17,** en sorte de former une première boucle **20,** de périmètre initial **P1** ajustable, dont au moins une portion **20a** est passée à travers les première **7** et seconde **8** ouvertures traversantes dudit élément d'appui **6.** La première extrémité libre **4** dudit élément longiligne **2** passe à travers ladite première ouverture traversante **7** dudit élément d'appui **6,** et ladite seconde extrémité libre **5** de l'élément longiligne **2** passe à travers la seconde ouverture traversante **8** dudit élément d'appui **6** et forme une deuxième boucle **22** de périmètre initial **P2** ajustable. Le manchon **17** recevant ladite portion **2a** d'élément longiligne **2** a un diamètre externe au repos **n0.** Dans cet exemple précis, **n0** est de l'ordre de 2,50 mm.

La distance **m0** et le diamètre externe **d0** dudit élément longiligne **2** sont déterminés en sorte qu'en fonctionnement, une traction exercée sur la première extrémité libre **4** provoque le rapprochement des orifices d'admission **15** et de sortie **16** et corrélativement l'expansion radiale du manchon **17.**

De préférence, la distance **m0** (mm) est supérieure à dix fois le diamètre externe **d0** de l'élément longiligne **2.** Dans cet exemple précis, la distance **m0** est de l'ordre de 40 mm.

Le périmètre initial **P2** de la seconde boucle **22** est supérieur au périmètre initial **P1**, de préférence le périmètre initial **P2** est supérieur ou égal à 1,5 fois le périmètre initial **P1.**

En fonctionnement, l'orifice de sortie **16** du manchon **17** étant disposée directement face à la première ouverture traversante **7** de l'élément d'appui **6** l'orifice de sortie **16** du manchon **17** vient en butée contre la première ouverture traversante **7.**

Tel que représenté sur les figures, la première boucle **20** est disposée à l'intérieur de la deuxième boucle **22,** et les première **20** et seconde **22** boucles s'étendent de la face d'appui **6a** dudit élément d'appui **6.**

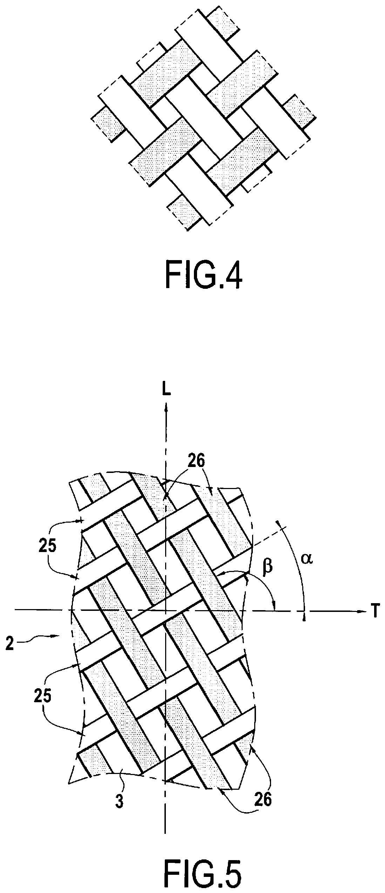

L'élément tubulaire longiligne **2** est une tresse tubulaire creuse de structure perlée (également désignée sous le terme de structure en diamant), représentée à la figure **4****,** obtenue par tricotage d'un premier groupe de mèches **25** tressées et d'un second groupe de mèches **26** tressées, le premier groupe de mèches **25** a une torsion **Z** tandis que le second groupe de mèches **26** a une torsion **S.** Sur le métier à tresser, chaque mèche est supportée individuellement sur un fuseau.

Chaque mèche du premier groupe de mèches **25** forme un angle **α** délimité entre l'axe longitudinal **L** de l'élément tubulaire longiligne **2** tressé et l'axe transversal **T,** sensiblement perpendiculaire à l'axe **L,** supérieur ou égal à +10° et inférieur ou égal à +90°, représenté à la figure **5****.** En parallèle, chaque mèche du second groupe de mèches **26** forme un angle **β** délimité entre l'axe longitudinal **L** de l'élément tubulaire longiligne **2** tressé et l'axe transversal **T,** supérieur ou égal à +100° et inférieur ou égal à +180°, également représenté à la figure **5****.**

Le premier groupe de mèches **25,** et le second groupe de mèches **26,** comprennent chacun huit mèches. Chaque mèche (indifféremment du premier groupe **25** ou du second groupe **26**) comprend six fils multifilamentaires, chacun de 138 dtex, retordus à environ 200 tours/mètre.

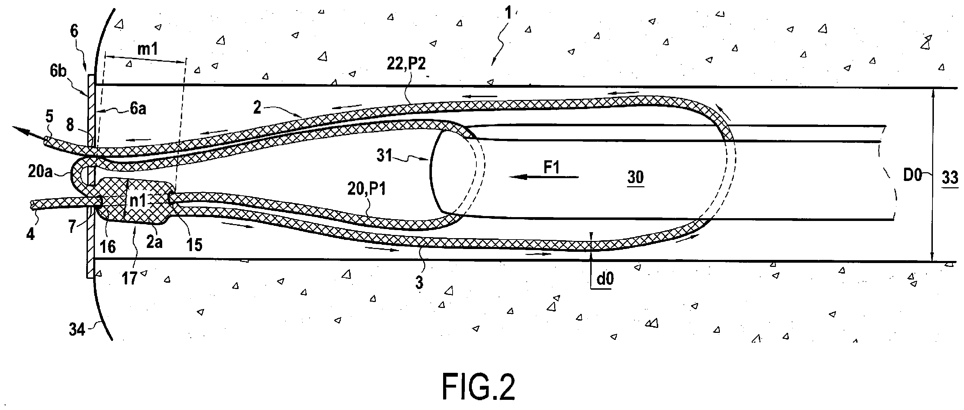

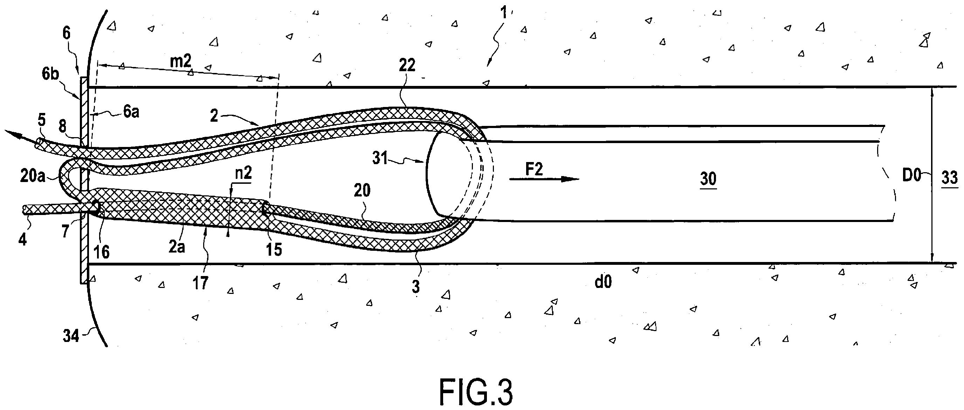

En fonctionnement, le chirurgien prépare le greffon **30** en sorte de former une boucle **31** dans ce dernier et passe cette boucle **31** dans les première **20** et seconde **22** boucles du dispositif implantable **1** puis dispose le dispositif implantable **1** solidarisé au greffon **30** dans au moins un canal osseux **33** ayant une section principale de diamètre interne constant **D0.** Un seul canal osseux **33** est représenté sur les figures **1** à **3** mais le greffon **30** pourrait être passé dans deux canaux osseux, tel que le canal tibial et le canal fémoral. Seule la boucle **31** du greffon **30** est représentée sur les figures **1** à **3** mais l'extrémité libre (non représentée) du greffon **30** peut être fixée à la paroi osseuse par n'importe quel moyen connu de l'état de la technique, tel que par exemple à l'aide d'un implant creux, logée dans ledit au moins un canal osseux **33,** recevant ladite extrémité bloquée par l'intermédiaire d'une vis vissée dans l'implant creux.

Une fois le greffon **30** passé dans ledit au moins un canal osseux **33,** la face d'appui **6a** de l'élément d'appui **6** étant en appui sur une paroi osseuse **34,** et les première **4** et seconde **5** extrémités libres de l'élément longiligne **2** se projetant de la face externe **6b** dudit élément d'appui **6,** à l'opposé de la face d'appui **6a,** le chirurgien exerce une traction sur la première extrémité libre **4** de l'élément longiligne **2,** selon une direction **F1,** provoquant la diminution du périmètre **P1** de la première boucle **20,** le rapprochement des orifices d'admission **15** et de sortie **16** du manchon **17** et corrélativement l'expansion radiale du manchon **17** adoptant un diamètre externe **n1** de l'ordre de 3 mm, la distance **m1** étant de l'ordre de 30 mm. Ces différents effets techniques permettent le réglage de la position du greffon **30** dans ledit au moins un canal osseux **33,** aidant ainsi à sa bonne mise en tension, et permettent l'immobilisation du manchon **17** dans ledit au moins un canal osseux **33** empêchant ainsi tout mouvement intempestif de l'élément longiligne **2** et donc corrélativement du greffon **30.**

L'immobilisation du dispositif implantable **1** est encore améliorée par le frottement exercée par l'orifice de sortie **16** du manchon **17** venant en butée contre la première ouverture traversante **7** de l'élément d'appui **6.**

Le chirurgien vient ensuite exercer une traction sur la seconde extrémité libre **5** de l'élément longiligne **2** entraînant l'extension du manchon **17** selon la direction **F2,** opposée à la direction **F1,** et donc l'éloignement des orifices d'admission **15** et de sortie **16** du manchon **17.** Le manchon **17** présente alors une distance **m2** supérieure à la distance **m0** ou **m1** et un diamètre externe **n2** inférieur aux diamètres **n0** et **n1.** Dans cet exemple précis, la distance **m2** est de l'ordre de 50 mm et le diamètre **n2** est de l'ordre de 2 mm. Cette position allongée du manchon **17** permet de bloquer le coulissement de l'élément longiligne **2** dans ledit manchon **17** et d'obtenir ainsi le verrouillage de l'élément longiligne **2** dans cette position bloquant ainsi la position du greffon **30** dans ledit au moins un canal osseux **33.** La traction sur la seconde extrémité libre **5** permet également de diminuer le périmètre **P2** de la seconde boucle **22** et d'ajuster encore éventuellement la position du greffon **30** dans ledit au moins un canal osseux **33.**

Le dispositif implantable **1** selon l'invention permet une immobilisation et blocage de l'élément longiligne **2** de manière réversible, il suffit en effet d'exercer une traction sur la première extrémité libre **4** ou la seconde extrémité libre **5** dans une direction opposée à la direction **F1** ou **F2,** tout en maintenant le manchon **17** afin de libérer l'élément longiligne **2** et obtenir de nouveau son coulissement dans le manchon **17.**

Le dispositif implantable **1** selon l'invention permet ainsi avantageusement d'immobiliser le greffon **30,** puis d'ajuster sa position dans au moins le canal osseux **33,** et enfin de bloquer le greffon dans sa position d'implantation, et ce de manière réversible sans endommager les tissus, le canal osseux ou le greffon à toute les étapes mises en oeuvre par le dispositif implantable **1.**

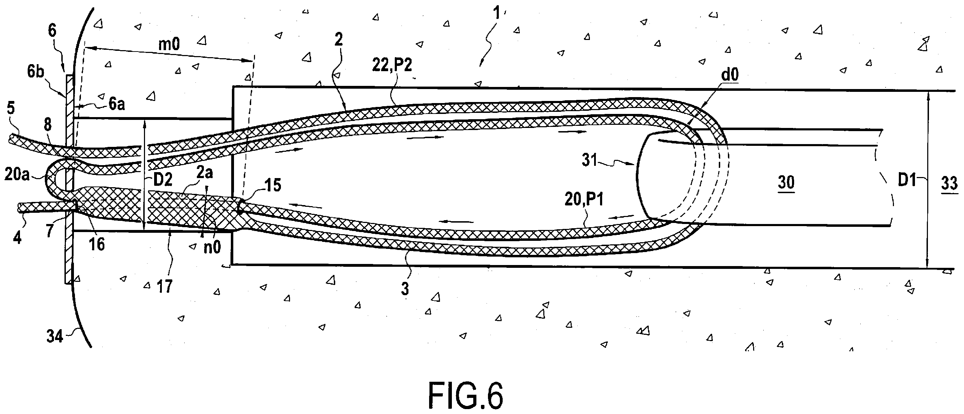

Le dispositif implantable **1** est représenté à la figure **6** dans un canal osseux présentant deux sections de diamètres internes différents, respectivement une première section de diamètre interne **D2** débouchant au niveau de l'élément d'appui **6** et recevant le manchon **17,** et une seconde section de diamètre interne **D1** recevant le greffon **30** passée dans les première **20** et seconde **22** boucles. Dans ce cas, lorsque le manchon **17** est à l'état comprimé, l'immobilisation du manchon **17** dans la section du canal osseux de plus faible diamètre **D2** est facilitée et encore améliorée comparativement au canal osseux représenté sur les figures **1** à **3** ayant une seule section de diamètre interne **D0.**

## Revendications

1. Dispositif de fixation implantable (1) pour la solidarisation d'un greffon à au moins un os d'une articulation, par exemple à l'os tibial et/ou à l'os fémoral, notamment pour la reconstruction du ligament croisé antérieur ou postérieur du genou, ledit dispositif de fixation comprenant :
- un élément textile longiligne tubulaire creux (2) délimitant un volume intérieur (3) et comprenant une première extrémité libre (4) et une seconde extrémité libre (5), et
- un élément d'appui (6) comprenant une face d'appui (6a) configurée pour venir en appui contre au moins ledit os d'une articulation, ledit élément d'appui (6) comprenant au moins deux ouvertures traversantes (7,8),
que ledit élément longiligne (2) a un diamètre externe initial (d0) (mm) et comprend un orifice d'admission (15) dans son volume intérieur et un orifice de sortie (16) de son volume intérieur (3) distants d'une distance initiale (m0) (mm) délimitant un manchon (17), la première extrémité (4) dudit élément longiligne (2) étant passée à travers lesdits orifices d'admission (15) et de sortie (16), dans le manchon (17), en sorte de former une première boucle (20), de périmètre initial (P1) ajustable, dont au moins une portion (20a) est passée à travers au moins deux ouvertures traversantes (7,8) dudit élément d'appui (6), la première extrémité libre (4) dudit élément longiligne (2) passe à travers une ouverture traversante (7) dudit élément d'appui (6), ladite seconde extrémité libre (5) passe à travers une ouverture traversante (8) dudit élément d'appui (6) et forme une deuxième boucle (22) de périmètre initial (P2) ajustable, le manchon (17), recevant une portion (2a) d'élément longiligne (2), a un diamètre externe au repos (n0), **caractérisé en ce que** la distance (m0) et le diamètre externe (d0) dudit élément longiligne (2) sont déterminés en sorte qu'en fonctionnement, une traction exercée sur la première extrémité libre (4) provoque le rapprochement des orifices d'admission (15) et de sortie (16) et corrélativement l'expansion radiale du manchon (17).

2. Dispositif de fixation (1) selon la revendication 1, **caractérisé en ce que** le périmètre initial (P2) est supérieur au périmètre initial (P1), de préférence le périmètre initial (P2) est supérieur ou égal à 1,5 fois le périmètre initial (P1).

3. Dispositif de fixation (1) selon l'une ou l'autre des revendications 1 et 2, **caractérisé en ce que** l'orifice de sortie (16) du manchon (17) vient en butée contre une ouverture traversante (7) dudit élément d'appui (6) lorsqu'en fonctionnement, une traction est exercée sur ladite première extrémité libre (4).

4. Dispositif de fixation (1) selon l'une quelconque des revendications 1 à 3, **caractérisé en ce que** lorsqu'une traction est exercée sur la seconde extrémité libre (5) dudit d'élément longiligne (2), les orifices d'admission (15) et de sortie (16) s'écartent pour atteindre une distance (m2) correspondant à une position de verrouillage dans laquelle la portion (2a) d'élément longiligne (2) disposée dans ledit manchon (17) est bloquée, en particulier la distance (m2) est supérieure à la distance initiale (m0) séparant lesdits orifices d'admission (15) et de sortie (16).

5. Dispositif de fixation (1) selon l'une quelconque des revendications précédentes, **caractérisé en ce qu'**une traction sur la seconde extrémité libre (5) entraîne la diminution du périmètre (P2) de la seconde boucle (22).

6. Dispositif de fixation (1) selon l'une quelconque des revendications précédentes, **caractérisé en ce que** la première boucle (20) est disposée à l'intérieur, au moins en partie, de la deuxième boucle (22), et **en ce que** les première (20) et seconde (22) boucles s'étendent de la face d'appui (6a) dudit élément d'appui (6).

7. Dispositif de fixation (1) selon l'une quelconque des revendications précédentes, **caractérisé en ce que** l'élément tubulaire longiligne (2) est une tresse.

8. Dispositif de fixation (1) selon l'une quelconque des revendications précédentes, **caractérisé en ce que** l'élément tubulaire (2) est une tresse de structure perlée.

9. Dispositif de fixation (1) selon l'une quelconque des revendications précédentes, **caractérisé en ce que** l'élément tubulaire longiligne (2) est une tresse comprenant un premier groupe de mèches tressées (25) et un second groupe de mèches tressées (26), le premier groupe de mèches (25) a une torsion (Z) tandis que le second groupe de mèches (26) a une torsion (S).

10. Dispositif de fixation (1) selon l'une quelconque des revendications précédentes, **caractérisé en ce que** l'élément tubulaire longiligne (2) est une tresse comprenant un premier groupe de mèches (25) dont chaque mèche forme un angle alpha délimité entre l'axe longitudinal (L) de l'élément tubulaire longiligne (2) tressé et l'axe transversal (T), sensiblement perpendiculaire à l'axe (L), supérieur ou égal à 10° et inférieur ou égal à 90°, et un second groupe de mèches (26) dont chaque mèche forme un angle beta délimité entre l'axe longitudinal (L) de l'élément tubulaire longiligne (2) tressé et l'axe transversal (T), sensiblement perpendiculaire à l'axe (L), supérieur ou égal à 100° et inférieur ou égal à 180°.

11. Dispositif (1) selon l'une ou l'autre des revendications 9 et 10, **caractérisé en ce que** le premier groupe de mèches (25), et éventuellement le second groupe de mèches (26), comprend/comprennent, au moins quatre mèches, encore de préférence au moins huit ou douze ou seize mèches.

12. Dispositif (1) selon quelconque des revendications 9 à 11, **caractérisé en ce que** chaque mèche du premier groupe de mèches (25), et éventuellement du second groupe de mèches (26), comprend/ comprennent au moins quatre fils, notamment au moins six fils, retordus selon un nombre de tours/mètre compris entre 100 et 300.

13. Dispositif (1) selon l'une quelconque des revendications 1 à 12, **caractérisé en ce que** l'élément tubulaire longiligne textile (2) comprend des fils ayant un titre supérieur ou égal à 100 dtex et inférieur ou égal à 300 dtex.

## Patentansprüche

1. Implantierbare Befestigungsvorrichtung (1) für die Verbindung eines Transplantats mit mindestens einem Knochen eines Gelenks, zum Beispiel mit dem Schienbeinknochen und/oder mit dem Oberschenkelknochen, besonders für die Rekonstruktion des vorderen oder hinteren Kreuzbands des Knies, wobei die Befestigungsvorrichtung umfasst:
- ein langgliedriges, rohrförmiges, hohles Textilelement (2), das ein inneres Volumen (3) begrenzt und ein erstes freies Ende (4) und ein zweites freies Ende (5) umfasst, und
- ein Auflageelement (6), umfassend eine Auflagefläche (6a), die dazu ausgestaltet ist, zumindest an dem Knochen eines Gelenks anzuliegen, wobei das Auflageelement (6) mindestens zwei Queröffnungen (7, 8) umfasst,
wobei das langgliedrige Element (2) einen ursprünglichen Außendurchmesser (d0) (mm) aufweist und in seinem inneren Volumen eine Einlassöffnung (15) und eine Auslassöffnung (16) seines inneren Volumens (3) umfasst, die in einem ursprünglichen Abstand (m0) (mm) beabstandet sind, der eine Muffe (17) begrenzt, wobei das erste Ende (4) des langgliedrigen Elements (2) durch die Einlass- (15) und die Auslassöffnung (16) in der Muffe (17) hindurchgeht, um eine erste Schlaufe (20) mit einem verstellbaren ursprünglichen Umfang (P1) zu bilden, von der mindestens ein Abschnitt (20a) durch mindestens zwei Queröffnungen (7, 8) des Auflageelements (6) hindurchgeht, wobei das erste freie Ende (4) des langgliedrigen Elements (2) durch eine Queröffnung (7) des Auflageelements (6) hindurchgeht, wobei das zweite freie Ende (5) durch eine Queröffnung (8) des Auflageelements (6) hindurchgeht und eine zweite Schlaufe (22) mit einem verstellbaren ursprünglichen Umfang (P2) bildet, wobei die Muffe (17), die einen Abschnitt (2a) des langgliedrigen Elements (2) aufnimmt, im Ruhezustand einen Außendurchmesser (n0) aufweist, **dadurch gekennzeichnet, dass** der Abstand (m0) und der Außendurchmesser (d0) des langgliedrigen Elements (2) so bestimmt werden, dass im Betrieb ein Zug, der auf das erste freie Ende (4) ausgeübt wird, die Annäherung der Einlass- (15) und der Auslassöffnung (16) und entsprechend die radiale Ausdehnung der Muffe (17) bewirkt.

2. Befestigungsvorrichtung (1) nach Anspruch 1, **dadurch gekennzeichnet, dass** der ursprüngliche Umfang (P2) größer als der ursprüngliche Umfang (P1) ist, vorzugsweise ist der ursprüngliche Umfang (P2) größer gleich dem 1,5-Fachen des ursprünglichen Umfangs (P1).

3. Befestigungsvorrichtung (1) nach einem der Ansprüche 1 und 2, **dadurch gekennzeichnet, dass** die Auslassöffnung (16) der Muffe (17) an einer Queröffnung (7) des Auflageelements (6) anliegt, wenn im Betrieb ein Zug auf das erste freie Ende (4) ausgeübt wird.

4. Befestigungsvorrichtung (1) nach einem der Ansprüche 1 bis 3, **dadurch gekennzeichnet, dass** sich, wenn ein Zug auf das zweite freie Ende (5) des langgliedrigen Elements (2) ausgeübt wird, die Einlass- (15) und die Auslassöffnung (16) voneinander entfernen, um einen Abstand (m2) zu erreichen, der einer Verriegelungsposition entspricht, in welcher der Abschnitt (2a) des langgliedrigen Elements (2), der in der Muffe (17) angeordnet ist, blockiert ist, wobei der Abstand (m2) insbesondere größer ist als der ursprüngliche Abstand (m0), der die Einlass- (15) und die Auslassöffnung (16) trennt.

5. Befestigungsvorrichtung (1) nach einem der vorhergehenden Ansprüche, **dadurch gekennzeichnet, dass** ein Zug auf das zweite freie Ende (5) die Verringerung des Umfangs (P2) der zweiten Schlaufe (22) mit sich bringt.

6. Befestigungsvorrichtung (1) nach einem der vorhergehenden Ansprüche, **dadurch gekennzeichnet, dass** die erste Schlaufe (20) zumindest teilweise im Inneren der zweiten Schlaufe (22) angeordnet ist, und dadurch, dass sich die erste (20) und zweite Schlaufe (22) von der Auflagefläche (6a) des Auflageelements (6) erstrecken.

7. Befestigungsvorrichtung (1) nach einem der vorhergehenden Ansprüche, **dadurch gekennzeichnet, dass** das langgliedrige, rohrförmige Element (2) ein Geflecht ist.

8. Befestigungsvorrichtung (1) nach einem der vorhergehenden Ansprüche, **dadurch gekennzeichnet, dass** das rohrförmige Element (2) ein Perlstrukturgeflecht ist.

9. Befestigungsvorrichtung (1) nach einem der vorhergehenden Ansprüche, **dadurch gekennzeichnet, dass** das langgliedrige, rohrförmige Element (2) ein Geflecht, umfassend eine erste Gruppe von geflochtenen Fasern (25) und eine zweite Gruppe von geflochtenen Fasern (26), ist, wobei die erste Gruppe von Fasern (25) eine Verdrehung (Z) aufweist, während die zweite Gruppe von Fasern (26) eine Verdrehung (S) aufweist.

10. Befestigungsvorrichtung (1) nach einem der vorhergehenden Ansprüche, **dadurch gekennzeichnet, dass** das langgliedrige, rohrförmige Element (2) ein Geflecht, umfassend eine erste Gruppe von Fasern (25), von denen jede Faser einen Winkel Alpha bildet, der zwischen der Längsachse (L) des geflochtenen langgliedrigen, rohrförmigen Elements (2) und der Querachse (T), im Wesentlichen senkrecht zu der Achse (L), begrenzt und größer gleich 10° und kleiner gleich 90° ist, und eine zweite Gruppe von Fasern (26) ist, von denen jede Faser einen Winkel Beta bildet, der zwischen der Längsachse (L) des geflochtenen langgliedrigen, rohrförmigen Elements (2) und der Querachse (T), im Wesentlichen senkrecht zu der Achse (L), begrenzt und größer gleich 100° und kleiner gleich 180° ist.

11. Vorrichtung (1) nach einem der Ansprüche 9 und 10, **dadurch gekennzeichnet, dass** die erste Gruppe von Fasern (25), und gegebenenfalls die zweite Gruppe von Fasern (26), mindestens vier Fasern, bevorzugter mindestens acht oder zwölf oder sechzehn Fasern, umfasst/umfassen.

12. Vorrichtung (1) nach einem der Ansprüche 9 bis 11, **dadurch gekennzeichnet, dass** jede Faser der ersten Gruppe von Fasern (25), und gegebenenfalls der zweiten Gruppe von Fasern (26), mindestens vier Fäden, insbesondere mindestens sechs Fäden, umfasst/umfassen, die gemäß einer Anzahl von Drehungen/Metern zwischen 100 und 300 zusammengedreht sind.

13. Vorrichtung (1) nach einem der Ansprüche 1 bis 12, **dadurch gekennzeichnet, dass** das langgliedrige, rohrförmige Textilelement (2) Fäden umfasst, die eine Feinheit größer gleich 100 dtex und kleiner gleich 300 dtex aufweisen.

## Claims

1. An implantable fastening device (1) for securing a graft to at least one bone of a joint, e.g. the tibial bone and/or the femoral bone, in particular for reconstructing the anterior or posterior cruciate ligaments of the knee, said fastening device comprising:
- a hollow tubular elongate textile element (2) defining an inside volume (3) and comprising a first free end (4) and a second free end (5); and
- a bearing element (6) having a bearing face (6a) configured to bear against at least said bone of a joint, said bearing element (6) comprising at least two through openings (7, 8);
said elongate element (2) has an initial outside diameter (d0) (mm) and comprises an admission orifice (15) leading into its inside volume and an exit orifice (16) going from its inside volume (3) that are spaced apart by an initial distance (m0) (mm) defining a sleeve (17), the first end (4) of said elongate element (2) being passed through said admission and exit orifices (15,16), into the sleeve (17), so as to form a first loop (20), of adjustable initial perimeter (P1), with at least a portion (20a) thereof being passed through at least two through openings (7, 8) of said bearing element (6), the first free end (4) of said elongate element (2) passes through a through opening (7) of said bearing element (6), said second free end (5) passes through a through opening (8) of said bearing element (6) and forms a second loop (22) of adjustable initial perimeter (P2), the sleeve (17) receiving a portion (2a) of elongate element (2) has an outside diameter at rest (n0), **characterized in that** the distance (m0) and the outside diameter (d0) of said elongate element (2) are determined in such a manner that in operation, traction exerted on the first free end (4) causes the admission and exit orifices (15,16) to move towards each other and correspondingly causes the sleeve (17) to expand radially.

2. A fastening device (1) according to claim 1, **characterized in that** the initial perimeter (P2) is greater than the initial perimeter (P1), with the initial perimeter (P2) preferably being greater than or equal to 1.5 times the initial perimeter (P1).

3. A fastening device according to claim 1 or claim 2, **characterized in that** the exit orifice (16) of the sleeve (17) comes into abutment against a through opening (7) of said bearing element (6) when, in operation, traction is exerted on said first free end (4).

4. A fastening device (1) according to any one of claims 1 to 3, **characterized in that** when traction is exerted on the second free end (5) of said elongate element (2), the admission and exit orifices (15,16) move apart so as to reach a distance (m2) corresponding to a locked position in which the portion (2a) of elongate element (2) placed in said sleeve (17) is blocked, in particular the distance (m2) is greater than the initial distance (m0) between said admission and exit orifices (15,16).

5. A fastening device (1) according to any one of the preceding claims, **characterized in that** traction on the second free end (5) causes the perimeter (P2) of the second loop (22) to decrease.

6. A fastening device 1) according to any one of the preceding claims, **characterized in that** the first loop (20) is arranged at least in part inside the second loop (22), and **in that** the first and second loops (20,22) extend from the bearing face (6b) of said bearing element (6).

7. A fastening device (1) according to any one of the preceding claims, **characterized in that** the elongate tubular element (2) is a braid.

8. A fastening device (1) according to any one of the preceding claims, **characterized in that** the tubular element (2) is a braid of pearl structure.

9. A fastening device (1) according to any one of the preceding claims, **characterized in that** the elongate tubular element (2) is a braid comprising a first group of braided strands (25) and a second group of braided strands (26), the first group of strands (25) having a (Z) twist while the second group of strands (26) has a (S) twist.

10. A fastening device(1) according to any one of the preceding claims, **characterized in that** the elongate tubular element (2) is a braid having a first group of strands (25) in which each strand forms an angle **α** between the longitudinal axis (L) of the braided elongate tubular element (2) and the transverse axis (T) that is substantially perpendicular to axis (L), which angle is greater than or equal to 10° and less than or equal to 90°, and a second group of strands (26) in which each strand forms an angle β defined between the longitudinal axis (L) of the braided elongate tubular element (2) and the transverse axis (T) that is substantially perpendicular to the axis (L), which angle is greater than or equal to 100° and less than or equal to 180°.

11. A fastening device (1) according to claim 9 or claim 10, **characterized in that** the first group of strands (25), and optionally the second group of strands (26) comprise(s) at least four strands, and more preferably at least eight (or twelve or sixteen) strands.

12. A fastening device (1) according to any one of claims 9 to 11, **characterized in that** each strand of the first group of strands (25), and optionally of the second group of strands (26), comprise(s) at least four yarns, in particular at least six yarns, that are twisted with a number of twists per meter lying in the range 100 to 300.

13. A fastening device (1) according to any one of claims 1 to 12, **characterized in that** the textile elongate tubular element (2) comprises yarns having a fineness greater than or equal to 100 dtex and less than or equal to 300 dtex.
